Europäisches Patentamt

European Patent Office

Office européen des brevets

(11) Numéro de publication: **0 093 033**
A1

## (12) DEMANDE DE BREVET EUROPEEN

(21) Numéro de dépôt: 83400720.5

(22) Date de dépôt: 12.04.83

(51) Int. Cl.³: **A 61 K 7/08**
A 61 K 7/06

(30) Priorité: 23.04.82 FR 8207031

(43) Date de publication de la demande:
02.11.83 Bulletin 83/44

(84) Etats contractants désignés:
AT BE CH DE GB IT LI LU NL SE

(71) Demandeur: Thorel, Jean-Noel
3 rue La Rochelle
F-75014 Paris(FR)

(72) Inventeur: Thorel, Jean-Noel
3 rue La Rochelle
F-75014 Paris(FR)

(74) Mandataire: Hirsch, Marc-Roger
34 rue de Bassano
F-75008 Paris(FR)

(54) Perfectionnements aux procédés de nettoyage et de restauration lipidique et produits pour leur mise en oeuvre.

(57) Procédé de nettoyage et de restauration de surfaces nécessitant la présence d'une couche lipidique protectrice présentant une première phase de lavage des lipides dégradés et des salissures par au moins un premier composé présentant au moins un premier radical lavant.

Produits pour la mise en oeuvre dudit procédé.

Application au nettoyage et à la restauration des peaux, cuirs, fourrures, cheveux, y compris sur êtres vivants.

EP 0 093 033 A1

PERFECTIONNEMENTS AUX PROCEDES DE NETTOYAGE ET DE
RESTAURATION LIPIDIQUE ET PRODUITS POUR LEUR MISE EN OEUVRE

La présente invention a pour objet des perfectionnements, aux procédés de nettoyage et d'entretien de toutes surfaces nécessitant la présence d'un film lipidique.

Elle a également pour objet les produits de régénération de toute surface ou de tout corps nécessitant la présence d'une couche lipidique de protection, ce qui est le cas de nombreux produits industriels tels que les cuirs, peaux et fourrures mais également des peaux notamment des peaux velues ou chevelues d'être vivants (hommes ou animaux).

Dans ce qui suit et à titre illustratif et par simplification on se réfèrera uniquement au cas le plus délicat du cuir chevelu.

L'expérience a montré que l'usage des shampooings et autres produits de nettoyage déclenche des anomalies du fait essentiellement de l'élimination de la couche protectrice lipidique. Sur le cheveu normal cette élimination des lipides peut entraîner une suractivité des glandes séba - cées par phénomène de contreréaction (feed back), ce qui se traduit par un surgraissage du cheveu ou par un engorgement des sécrétions à la base de la tige. Il en résulte donc un cheveu par trop sec.

Ces situations opposées et extrêmes sont aussi nocives l'une que l'autre et entraînent des effets secondaires se traduisant par un désordre et un déséquilibre biologique du cheveu, pouvant entraîner une fragilisation et même l'alopécie.

L'art antérieur a donc tenté d'éviter ces inconvénients en mettant sur le marché des shampooings à effet réduit par une simple dilution,ce qui présente l'inconvénient de réduire les effets nettoyants dans les mêmes proportions que les effets nocifs.

On a également proposé des produits à base d'écorce de quillaya et toute question d'incidence sur la permanence du film lipidique mise à part,

2 0093033

on constate qu'ils ne moussent pas et surtout sont dangereux pour les yeux.

Or, il est évident que l'hygiène la plus élémentaire nécessite l'élimination des salissures fixées aux cheveux et au cuir chevelu, des débris dermiques, et des graisses dégénérées ou décomposées sur lesquelles se sont fixés les éléments polluants atmosphériques et les bactéries.

L'invention a pour but d'éliminer tout ce qui doit être éliminé et de restaurer le film lipidique. Pour ce faire, il fallait réunir les propriétés suivantes: une non agressivité, la biodégradabilité, la propreté bactériologique, la non délipidation, la mise en émulsion des graisses dégénérées, l'évacuation des salissures, c'est-à-dire un lavage non dégradant mais restaurant.

La présente invention résulte d'une analyse approfondie des phénomènes entraînés par les détergents et autres produits traditionnels, sur un plan physicochimique et sur un plan biologique très étendu.

La solution retenue, conformément à l'invention, combine de façon imprévue au moins un radical de préférence non ionique, peu délipidant, à effet de surface et éventuellement mouillant avec au moins un radical lipidique et éventuellement avec au moins un radical non ionique émulsionnant et éventuellement également avec au moins un radical bactéricide et un radical protéinique

Les expériences ont démontré que le cheveu après traitement présente une couche propre et régulière de lipides assurant une protection normale au cheveu et évitant une hyperséborrhée.

On notera également que ceci permet le lavage des cheveux des sujets atteints d'eczéma, de psoriasis ou d'autres maladies qui ne peuvent supporter l'action irritante des shampooings.

Ceci ne peut être obtenu que dans des dosages ou proportions précises qui n'ont pu être déterminés que grâce aux méthodes de contrôle et d'analyse mis au point par la demanderesse.

Dans ce qui suit et par simplification on va se référer à des radicaux actifs et, en ce qui concerne les proportions pondérales, aux molécules actives correspondantes étant bien entendu comme on l'a souligné plus haut que comme dans de nombreuses applications les radicaux jouent un rôle prépondérant en rendant la plupart du temps négligeable celui de la molécule à laquelle peuvent se rattacher un ou plusieurs radicaux d'un ou plusieurs types différents (oxyde d'éthylène, ou ses dérivés, tel polyoxéthyléne, par exemple). Ce genre d'estércification ou de réaction équivalente peut être effectuée par tout moyen désormais classique.

La base de l'invention repose sur les phases d'un procédé de restauration de corps et de surfaces nécessitant la présence d'une couche protectrice lipidique, ce qui s'applique tout particulièrement aux surfaces cutanées et notamment au cuir chevelu et aux cheveux.

Un premier radical (type 1) à caractère lavant induit une micro-émulsion agissant notamment sur les lipides dégradés, ce qui a pour effet de mobiliser les salissures fixées par la couche lipidique dégradée. Par addition de la phase aqueuse de lavage au moins un second radical (type 2) crée une émulsion de la phase aqueuse et de la phase lipidique. Vont se produire alors à la fois une élimination des salissures et des lipides dégradés et un dépôt en surface d'une émulsion qui va inclure les lipides intacts restants et au moins un troisième type de radical lipidique (type 3).

Au séchage la phase aqueuse disparaît laissant un film lipidique protecteur, propre et régénéré. C'est du choix des radicaux que vont dépendre les effets imprévisibles de l'invention, c'est-à-dire une substitution des salissures et des produits résultant de la dégradation des lipides par les lipides de régénération venant se mélanger et/ou se combiner avec les lipides non dégénérés de la couche protectrice traitée.

Selon un mode de réalisation préféré de l'invention, on peut y associer au moins un radical protéinique d'un quatrième type (type 4) qui assure à la fois nourriture et l'aspect brillant à la couche lipidique, ce qui a ici d'autant plus d'importance que par phénomène de diffraction la présence de lipides modifie l'aspect de brillance.

On peut également selon un autre mode de réalisation préféré de l'invention améliorer l'effet des deux premiers types de radicaux en renforçant l'effet d'élimination des salissures et l'effet d'assainissement grâce à au moins un radical d'un cinquième type à effet bactéricide (type 5).

C'est du dosage de ces divers radicaux que dépend la qualité du traitement et de l'état final de la surface. On remarquera que les deux premiers types de radicaux peuvent se confondre ou être différents.

Pour mieux faire comprendre les caractéristiques techniques et les avantages de la présente invention, on va en décrire des exemples de réalisation étant bien entendu que ceux-ci ne sont pas limitatifs quant à leur mode de mise en oeuvre et aux appplications qu'on peut en faire.

L'ensemble des exemples ci-dessous a donné lieu à des essais en milieu hospitalier, en salon de coiffure, en clientèle pharmaceutique ainsi que sur divers sujets (notamment sur animaux pour les tests classiques d'irritation cutanée et oculaire et de toxicité orale). La quasi totalité de ces tests se sont montrés très satisfaisants quant aux effets recherchés et dans les proportions ci-dessous revendiquées, on n'a rencontré au-

4     0093033

cun problème majeur sur les plans des effets négatifs (toxicité, irritation, allergénicité, etc...).

Les compositions ci-dessous ne mentionnent que sommairement d'autres produits dont le rôle est classique: parfums, stabilisants, conservateurs, éventuellement lipoprotéines... dont la somme des teneurs en poids
correspond à la quantité suffisante pour atteindre 100%.

Lorsque les molécules ou les radicaux actifs le permettent ou le
nécessitent, on prépare séparément deux phases dont une lipidique puis on
les associe, ce qui est facilité par la présence de radicaux à effet émulsionnant. Les teneurs pondérales sont indiquées indépendamment des interactions ou combinaisons pouvant intervenir comme il a été indiqué ci-dessus
préalablement ou pendant la préparation des produits finaux.

Les radicaux des types 1 et 2 sont non ioniques. L'ensemble doit
être non agressif vis-à-vis des corps et surfaces à traiter. Pour le cuir
chevelu dont le pH est de l'ordre de 5,5 à 6, on a donc intérêt à être
légèrement acide. La plupart des exemples ci-dessous ont été réalisés à
des pH de l'ordre de 5,5.

De plus, conformément à l'invention l'ensemble doit être de préférence délipidant et biodégradable. On respectera de préférence les proportions pondérales suivantes:

$$\frac{\text{radicaux de type 2}}{\text{radicaux de type 1}} = 0 \text{ à } 45\%$$

$$\frac{\text{radicaux de type 3}}{\text{radicaux de type 1}} = 15 \text{ à } 70\%$$

$$\frac{\text{radicaux de type 4}}{\text{radicaux de type 1}} = 0 \text{ à } 6\%$$

$$\frac{\text{radicaux de type 5}}{\text{radicaux de type 1}} = 0 \text{ à } 3\%$$

Exemple 1:
- lanoline éthoxylénée ................................................ 13%
- alcool oléique polyoxyéthyléné 20 ........................... 2,5%
- monodiéthanolamine d'acide oléique ........................ 4,5%
- hydrolysat de protéines ...................................... 0,5%
- méthylisothiazolinone ...................................... 0,2%
- eau déminéralisée et excipients divers q.s.p. ............à 100%

Exemple 2:
- monoditristéarate de sucrose acetylé ..................... 15%
- monophénol polyoxyéthyléné 10 ......................... 4%
- oxyde d'alkyl amidopropyl diméthylamine ................... 6%

- hydrolisat de protéine .................................... 0,75%

- méthyl chloroisothiazolinone ............................. 0,25%

- eau déminéralisée et excipients divers q.s.p. ............à 100%

Exemple 3:

- octyl glucoside ............................................ 15%

- huile de ricin polyoxyéthylénée 40 ........................ 3%

- glycéride d'acide caprique ................................ 7,5%

- hydrolysat de protéine .................................... 0,5%

- phénoxyéthanol ........................................... 0,25%

- eau déminéralisée et excipients divers q.s.p. ............à 100%

Exemple 4:

- décylglucoside ............................................ 14%

- alcool cétylique polyoxyéthyléné  20 ...................... 2,5%

- glycéride d'acide caprylique .............................. 5,5%

- hydrolisat de protéine .................................... 0,4%

- phénoxyéthanol ........................................... 0,2%

- eau déminéralisée et excipients divers q.s.p. ............à 100%

Exemple 5:

- éther cétastéanylique polyoxyéthyléné  .................... 15%

- monolaurate de sorbitan polyoxyéthyléné 20 ................ 3,5%

- érucylate d'oléyle ........................................ 5%

- hydrolysat de protéine .................................... 0,6%

- phénoxyéthanol ........................................... 0,25%

- eau déminéralisée et excipients divers q.s.p. ............à 100%

Chacun des exemples ci-dessus a donné lieu à de nombreux assais résumés comme suit, les marges de pourcentages correspondant aux divers produits:

Essais sur l'homme

- bonne tolérance oculaire ..................... 92 à 98%

- bonne tolérance du cuir chevelu .............. 97 à 99%

- pouvoir nettoyant bon ........................ 60 à 66%

- pouvoir nettoyant normal ..................... 30 à 34%

Les cas les moins favorables n'ont présenté aucun signe à conséquence appréciable.

Etude bactériologique:

Le pouvoir inhibiteur intrinsèque peut être considéré comme excellent sur les souches suivantes essayées expérimentalement staphylococcus aureus, klebsiella pneumoniae, saccharomyces cerevisiae. On a trouvé au contrôle bactériologique ni germes, ni bactéries, ni levures ou moisissures.

### Essais sur animaux:

Les essais d'irritation oculaire et cutanée chez le lapin ont été très satisfaisants sur le rat. Les tests de pouvoir sensibilisant par application locale sur le cobaye n'ont révélé aucune allergie.

En fait tous les produits ont donné d'excellents résultats sur le plan pratique hygiénique, cosmétique, capillaire et dermatologique.

De très nombreux essais ont été également menés avec d'autres radicaux ou molécules appartenant aux mêmes grands types de produits: parmi les non ioniques surfactifs on peut citer: cocoate de sucrose, monodistéarate de sucrose, éther cétastéarylique polyoxyéthyléné, éther laurique polyoxyéthyléné, oxyde de stéaryldiméthylamine, oxyde de lauryl diméthylamine.

Parmi les lipidants on peut citer: toutes les huiles simples substantivées: cétéarylacétonate, oléate de décyle, monodiéthanolamines d'acides gras, associations acide linoléique/acide linoléique faiblement quaternisé.

On a essayé également avec satisfaction des combinaisons des divers radicaux et molécules ci-dessus énumérés notamment dans les exemples.

On remarquera que lors des essais on a pu constater sur les divers types de cheveux (gras et secs notamment) une restauration de la couche lipidique aux teneurs des cheveux normaux.

Dans tous les cas on a gardé les performances cosmétiques généralement recherchées, notamment du point de vue du coiffage, de la brillance et du moussage.

Il est précisé que le caractère non ionique correspond à un mode de réalisation préféré de l'invention. On peut en fait utiliser dans le cadre de l'invention des cations, des anions ou des amphotères mais dans ces cas il est nécessaire d'augmenter considérablement la proportion des surlipidants (type 3) ce qui peut dans certains cas augmenter le prix de revient pour des effets non supérieurs.

On notera enfin que tous les essais menés sur matériaux non vivants (peaux, cuirs, fourrures, métaux huilés, etc...) se sont révélés particulièrement satisfaisants.

Bien entendu, l'invention n'est pas limitée aux modes de réalisation décrits et représentés et elle est susceptible de nombreuses variantes accessibles à l'homme de l'art, sans que l'on ne s'écarte de l'esprit de l'invention.

REVENDICATIONS

1.- Procédé de nettoyage et de restauration de surfaces nécessitant la présence d'une couche lipidique protectrice comportant une première phase de lavage des lipides dégradés et des salissures par au moins un premier composé présentant au moins un premier radical lavant caractérisé par le fait que dans une seconde phase au moins un second composé présentant au moins un second radical émulsifiant crée avec l'eau ajoutée une émulsion phase aqueuse/phase lipidique, que dans une troisième phase au moins un troisième composé présentant au moins un troisième radical lipidique laisse la couche lipidique restaurée après élimination de l'eau de lavage émulsifiée.

2.- Procédé selon la revendication 1, caractérisée par le fait que dans une quatrième phase au moins un quatrième composé, présentant au moins un quatrième radical protéinique est apporté, qui restera sur la surface avec la couche lipidique régénérée.

3.- Procédé selon l'une des revendications 1 ou 2, caractérisé par le fait que la couche régénérée est obtenue après un séchage final.

4.- Procédé selon l'une des revendications 1 à 3, caractérisé par le fait qu'au moins un cinquième composé présentant au moins un cinquième radical bactéricide est apporté au traitement de la surface.

5.- Procédé selon l'une des revendications 1 à 4, caractérisé par le fait que tout ou partie des premiers, seconds, troisièmes, quatrièmes et cinquièmes composés sont confondus.

6.- Produit de nettoyage et de restauration des surfaces nécessitant la présence d'une couche lipidique protectrice caractérisé par le fait qu'il comprend les éléments constitutifs définis aux revendications 1 à 5.

7.- Produit selon la revendication 6, caractérisé par le fait que la proportion pondérale entre molécules équivalentes des seconds radicaux et premiers radicaux est de 0 à 45%.

8.- Produit selon l'une des revendications 6 ou 7, caractérisé par le fait que la proportion pondérale entre molécules équivalentes des troisièmes radicaux et premiers radicaux est de 15 à 70%.

9.- Produit selon l'une des revendications 6 à 8, caractérisé par le fait que la proportion pondérale entre molécules équivalentes des quatrièmes radicaux et premiers radicaux est de 0 à 6%.

10.- Produit selon l'une des revendications 6 à 9, caractérisé par le fait que la proportion pondérale entre molécules équivalentes des cinquièmes radicaux et premiers radicaux est de 0 à 3%.

0093033

11.- Produit selon l'une des revendications 6 à 10, caractérisé par le fait que tout ou partie des radicaux sont non ioniques.

12.- Produit selon l'une des revendications 6 à 11, caractérisé par le fait que tout ou partie des radicaux sont éthoxylénés.

13.- Produit selon l'une des revendications 6 à 12, caractérisé par le fait que tout ou partie des radicaux sont polyéthoxylénés.

14.- Application des procédé et produit selon une quelconque des revendications 1 à 13 au traitement de peaux.

15.- Application des procédé et produit selon la revendication 14, caractérisée en ce que les peaux sont celles d'êtres vivants à peau velue.

16.- Application des procédé et produit selon la revendication 14, caractérisée en ce que les peaux sont des peaux, cuirs et fourrures non vivants.

Office européen
des brevets

**RAPPORT DE RECHERCHE EUROPEENNE**

EP  83  40  0720

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X,Y | FR-A-2 401 187  (L'OREAL)<br><br>*Revendication;        examples A1,A2,A3,A4;  page  17, ligne 5 - page 18, ligne 35*<br>--- | 1,3,5, 6-8,11 ,12-15 | A 61 K    7/08<br>A 61 K    7/06 |
| X,Y | FR-A-2 358 877  (COLGATE)<br><br>*Revendication; example; page 13, ligne 6 - page 14, ligne 32*<br>--- | 1,3,5- 8,11- 15 | |
| X,Y | FR-A-2 416 009  (FREUDENBERG)<br><br>*Revendication;    example  3; page 4, lignes 6-16*<br>--- | 1-3,5- 9,11- 15 | |
| X,Y | US-A-3 666 857  (K.L.RUSSELL)<br><br>*Revendication;   example; colonne 1, ligne 53 - colonne 3, ligne 6*<br>--- | 1,3,5- 8,11- 15 | DOMAINES TECHNIQUES RECHERCHES (Int. Cl. ³)<br><br>A 61 K    7/08<br>A 61 K    7/06 |
| Y | SEIFEN-ÖLE-FETTE-WACHSE, vol. 106, no. 11, 3 juillet 1980, pages 301-303, Augsburg DE; G.LUDWIG:  "Aufbau, Eigenschaften und  Andwendung  von  Emulgatoren für Kosmetika" *En entier*<br>----- | 1,3,5, 11-15 | |

Le présent rapport de recherche a été établi pour toutes les revendications

| Lieu de la recherche<br>LA HAYE | Date d'achèvement de la recherche<br>31-05-1983 | Examinateur<br>VANHECKE H. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82